# EUROPÄISCHE PATENTSCHRIFT

(11) **EP 2 871 620 B1**
(45) Veröffentlichungstag und Bekanntmachung des Hinweises auf die Patenterteilung: **03.01.2018**
(21) Anmeldenummer: 14191904.3
(22) Anmeldetag: 05.11.2014
(51) Int. Cl.: G08B 17/10, G01N 1/26, G08B 17/107, G08B 29/00, B08B 5/02, G01N 15/00, B08B 9/00, G01N 33/00, G08B 17/113

(54) **Rauchdetektionsanordnung**
Smoke detection assembly
Agencement de détection de fumée

(30) Priorität: 07.11.2013 AT 8542013
(43) Veröffentlichungstag der Anmeldung: 13.05.2015
(73) Patentinhaber: Labor Strauss Sicherungsanlagenbau Ges. m. b. H, 1231 Wien (AT)
(72) Erfinder: Friedl, Stefan, 1231 Wien (AT)
(74) Vertreter: Puchberger & Partner Patentanwälte

(56) Entgegenhaltungen:
- WO-A1-2004/053331
- DE-U1-202008 006 811
- GB-A- 2 243 475

## Beschreibung

Die Erfindung betrifft eine Rauchdetektionsanordnung zur Detektion von Rauchgas oder Rauchpartikeln in der Luft eines Gebäudes, umfassend ein Ansaugsystem zur Erzeugung eines Unterdrucks, eine Ansaugleitung über die das Rohrleitungssystem mit dem Ansaugsystem verbunden ist, eine Detektionsvorrichtung, die mit dem Ansaugsystem und/oder der Ansaugleitung verbunden ist, um Rauchgas in dem aus dem Rohrleitungssystem angesaugten gasförmigen Medium zu detektieren, eine Freiblasvorrichtung mit einer Überdruckquelle zur Einleitung eines gasförmigen Mediums in die Ansaugleitung, wobei ein Ventil vorgesehen ist, über das eine Verbindung der Überdruckquelle mit der Ansaugleitung geöffnet oder geschlossen werden kann.

Rauchdetektionsanordnungen sind seit langer Zeit bekannt und in unterschiedlichen Ausführungsformen dem Stand der Technik zu entnehmen.

Für die Überwachung von Gebäuden kommen Rauchdetektionsanordnungen zum Einsatz, bei denen über ein Rohrleitungssystem dem Raum Proben entnommen werden, die einer Detektionsvorrichtung zugeführt werden. Diese Detektionsvorrichtung ist dazu eingerichtet, Bestandteile des gasförmigen Mediums, insbesondere der Luft, zu analysieren, um Rauchgase aufzuspüren. So kommt es zu einer Früherkennung eines etwaigen Brandes in dem Gebäude.

Das Dokument DE 2008 006811 U1 weist die Benutzung mehreren Ventilen pro Leitung aus, die separat und mit viel technischen Aufwand angesteuert werden. Bevorzugt werden gemäß Stand der Technik Rauchdetektionsanordnungen eingesetzt, die kontinuierlich einen Teilmassenstrom aus dem Gebäude ansaugen und diesen der Detektionsvorrichtung zuführen. Das angesaugte Medium enthält neben Luft auch Partikel wie beispielsweise Staub, die sich in weiterer Folge in der Rauchdetektionsanordnung ablagern und im schlechtesten Fall die Funktion der Detektionsvorrichtung beeinträchtigen. Aus diesem Grund werden vor der Detektionsvorrichtung Filter eingesetzt, die das Eindringen von Staub in die Detektionsvorrichtung verhindern sollen. Jedoch tritt ein weiteres Problem auf, nämlich, dass die Ansaugleitung, die den Teilmassenstrom zur Detektionsvorrichtung leitet durch diese Partikel verlegt wird. Aus diesem Grund kommen Freiblasvorrichtungen zum Einsatz.

Freiblasvorrichtungen umfassen eine Überdruckquelle wie beispielsweise einen Kompressor oder einen Pufferspeicher, aus der ein gasförmiges Medium stoßartig in die Ansaugleitung abgegeben wird, um diese freizublasen. Zum Schutz der Detektionsvorrichtung muss die Ansaugleitung von der Detektionsvorrichtung getrennt werden, um zu vermeiden, dass die Druckluft bzw. der Druckstoß die Detektionsvorrichtung beschädigt. Dies wird gemäß Stand der Technik mit einem Magnetventil ausgeführt, das die Ansaugleitung von der Detektionsvorrichtung trennt. Um die Überdruckquelle mit der Ansaugleitung zu verbinden, ist ein weiteres Magnetventil notwendig. Ferner ist eine Steuerung notwendig, die in einem ersten Schritt das Magnetventil zum Schutz der Detektionsvorrichtung schließt und in einem zweiten Schritt die Verbindung der Überdruckquelle mit der Ansaugleitung öffnet, um das Freiblasen zu bewirken. Zur Steuerung dieser Ventile sind gemäß Stand der Technik einfache Steuerungsvorrichtungen vorgesehen. Nachteil dieser Konstruktion ist, dass zwei Magnetventile sowie eine Steuerung vorgesehen sein müssen. Gattungsgemäße Rauchdetektionsanordnungen sind meist an schwer zugänglichen Orten angeordnet an denen darüber hinaus wenig Platz zur Verfügung steht. Ferner müssen diese Detektionsanordnungen bevorzugt wartungsfrei und zuverlässig funktionieren.

Aufgabe der Erfindung ist es nun, eine Rauchdetektionsanordnung zu schaffen, die zuverlässig und im Wesentlichen wartungsfrei arbeitet, und die darüber hinaus günstig in der Herstellung und kompakt ist.

Die erfindungsgemäße Aufgabe wird insbesondere durch die Merkmalskombination des unabhängigen Patentanspruchs gelöst.

Weitere vorteilhafte Merkmale der Rauchdetektionsanordnung zur Detektion von Rauchgas und/oder Rauchpartikeln in der Luft eines Gebäudes, umfassend ein Ansaugsystem zur Ansaugung von Luft über ein Rohrleitungssystem, eine Ansaugleitung, über die das Rohrleitungssystem mit dem Ansaugsystem verbunden ist, eine Detektionsvorrichtung, die mit dem Ansaugsystem und/oder der Ansaugleitung verbunden ist, um Rauchgas in der aus dem Rohrleitungssystem angesaugten Luft zu detektieren, eine Freiblasvorrichtung mit einer Überdruckquelle zur Einleitung eines gasförmigen Mediums in die Ansaugleitung, wobei ein Ventil vorgesehen ist, über das eine Verbindung der Überdruckquelle mit der Ansaugleitung geöffnet oder geschlossen werden kann, sind, dass das Ventil eine Ansaugstellung aufweist, in der die Detektionsvorrichtung mit dem Ansaugsystem und der Ansaugleitung verbunden ist, dass das Ventil eine Freiblasstellung aufweist, in der die Detektionsvorrichtung von der Ansaugleitung getrennt ist und in der die Überdruckquelle mit der Ansaugleitung verbunden ist, und dass, wenn ein Überdruck von der Überdruckquelle auf das Ventil wirkt, das Ventil, angetrieben vom gasförmigen Medium der Überdruckquelle, selbsttätig von der Ansaugstellung in die Freiblasstellung wechselt.

Ferner kann die Erfindung derart ausgestaltet sein, dass das Ventil zwischen der Freiblasstellung und der Ansaugstellung eine Sperrstellung aufweist, in der die Überdruckquelle, die Detektionsvorrichtung und die Ansaugleitung voneinander getrennt sind, dass das Ventil einen Kolben aufweist, der in einem Ventilgehäuse von der Ansaugstellung in die Freiblasstellung bewegbar ist, dass das Ventilgehäuse eine Kolbenöffnung aufweist, deren Profil im Wesentlichen dem Profil des Kolbens entspricht, sodass dieser in der Kolbenöffnung geführt ist und dass bevorzugt der Kolbenspalt zwischen Kolbenöffnung und Kolben im Wesentlichen abgedichtet ist, wobei der Kolben im Ventilgehäuse gegebenenfalls linear geführt ist, dass der Kolben einen Lüftungskanal umfasst, der im Wesentlichen quer zur Bewegungsachse verläuft und an einer ersten Stelle der Mantelfläche in den Kolben eintritt und an einer zweiten Stelle der Mantelfläche austritt, sodass der Kolben durch den Lüftungskanal durchsetzt ist, dass das Ventilgehäuse eine erste Lüftungsöffnung und eine zweite Lüftungsöffnung umfasst und dass in der Ansaugstellung des Ventils die erste Lüftungsöffnung über den Lüftungskanal des Kolbens mit der der zweiten Lüftungsöffnung verbunden ist, dass der Kolben einen Freiblaskanal umfasst der sich von der ersten Stirnseite des Kolbens bis zu einer dritten Stelle der Mantelfläche des Kolbens erstreckt, dass das Ventilgehäuse eine Freiblasöffnung umfasst, und/oder dass in der Freiblasstellung des Ventils die Freiblasöffnung über den Freiblaskanal des Kolbens mit der ersten Lüftungsöffnung verbunden ist.

Ferner kann die Erfindung derart ausgestaltet sein, dass zwischen dem Lüftungskanal an der ersten Stelle der Mantelfläche des Kolbens und dem Freiblaskanal an der dritten Stelle der Mantelfläche des Kolbens ein Steg vorgesehen ist, durch welchen die erste Lüftungsöffnung in der Sperrstellung, insbesondere beim Übergang von der Ansaugstellung in die Freiblasstellung, verschlossen ist, dass an der zweiten Stirnseite des Kolbens ein Druckmittel, bevorzugt ein elastischer Körper wie beispielsweise eine Druckfeder, vorgesehen ist, die insbesondere in der Freiblasstellung gegen den die zweite Stirnseite des Kolbens wirkt, und dass die Druckkraft des Druckmittels in der Ansaugstellung geringer ist, als die durch die Überdruckquelle auf die erste Stirnseite wirkende Druckkraft, sodass sich der Kolben bei angelegter Druckkraft der Überdruckquelle in die Freiblasstellung bewegt und/oder in der Freiblasstellung befindet, und/oder dass der auf die erste Stirnseite des Kolbens wirkende Druck des gasförmigen Mediums der Überdruckquelle größer ist, als der Druck des gasförmigen Mediums in der Freiblasleitung, dass der Druck des gasförmigen Mediums zum Freiblasen der Ansaugleitung im Ventil oder beim Austritt aus dem Ventil größer ist, als der herrschende Luftdruck in der Ansaugleitung, sodass das gasförmige Medium der Überdruckquelle zum Freiblasen der Ansaugleitung gegen die Ansaugrichtung in die Ansaugleitung und gegebenenfalls in das Rohrleitungssystem geleitet ist und/oder dass das Ventil und/oder der Kolben von der Freiblasstellung selbsttätig in die Ansaugstellung zurückkehrt, wenn die auf die erste Stirnseite des Kolbens wirkende Druckkraft kleiner ist, als die Kraft des Druckmittels.

Weiters kann die Erfindung derart ausgestaltet sein, dass das Ansaugsystem kontinuierlich Luft und gegebenenfalls in der Luft enthaltenes Rauchgas in die Detektionsvorrichtung fördert, um eine kontinuierliche Rauchgasdetektion zu ermöglichen, dass im Ansaugbereich der Detektionsvorrichtung ein Filter, eine Trocknungsvorrichtung, eine Kühlvorrichtung oder ähnliche Komponenten vorgesehen sind, dass die in die Detektionsvorrichtung geleitete Luft über eine Rückführungsleitung wieder zurück in das Rohrleitungssystem geleitet ist, dass die Komponenten Ventil, Detektionsvorrichtung und/oder Ansaugsystem als Einheit in einem Gehäuse angeordnet sind, und/oder dass der Kolben als runder, insbesondere zylindrischer Kolben ausgeführt ist, der entlang seiner Drehachse bewegbar geführt, jedoch gegen eine Verdrehung gesichert ist.

Die erfindungsgemäße Vorrichtung oder Anordnung ist gegebenenfalls derart ausgestaltet, dass sich ein Rohrleitungssystem in unterschiedliche Bereiche und/oder Räume eines Gebäudes erstreckt, dass über oder durch dieses Rohrleitungssystem Luftproben aus unterschiedlichen Bereichen des Gebäudes entnommen und an ein zentrales Detektionssystem geleitet werden und/oder dass das Rohrleitungssystem gegebenenfalls verzweigt ausgestaltet ist, wobei sich bevorzugt einzelne Zweige des Rohrleitungssystem in unterschiedliche Bereiche und/oder Räume eines Gebäudes erstrecken. Gegebenenfalls entspricht das Rohrleitungssystem den Leitungen eines zentralen Lüftungssystems oder einem eigens zur Detektion von Rauchbestandteilen eigerichteten Rohrleitungssystem.

Gegebenenfalls ist vorgesehen, dass an der ersten Stirnseite des Kolbens eine entlang der Bewegungsachse in den Kolben verlaufende Ausnehmung oder Bohrung vorgesehen ist, die zumindest einen Teil des Freiblaskanals bildet, dass ein Freiblasrohr vorgesehen ist, das sich vom Gehäuse entlang der Bewegungsachse in die Kolbenöffnung erstreckt und an dessen freien Ende die Freiblasöffnung vorgesehen ist, sodass von der Überdruckquelle kommendes gasförmigen Mediums durch das Freiblasrohr und die Freiblasöffnung Richtung Kolben geleitet werden kann, und dass das Freiblasrohr in der Ansaugstellung in die Ausnehmung ragt.

Die erfindungsgemäße Rauchdetektionsanordnung weist ein Ansaugsystem mit einer Ansaugleitung auf. Das Ansaugsystem ist dazu geeignet und/oder eingerichtet einen Unterdruck zu erzeugen und/oder das gasförmige Medium bzw. die Luft des Gebäudes zu der Detektionsvorrichtung zu fördern. Dazu ist die Detektionsvorrichtung über die Ansaugleitung mit dem Rohrleitungssystem verbunden.

Gegebenenfalls sind im Bereich vor der Detektionsvorrichtung weitere Komponenten wie beispielsweise ein Filter, eine Trocknungsvorrichtung und/oder weitere Einrichtungen zur Aufbereitung des zu detektierenden gasförmigen Mediums vorgesehen. Das Ansaugsystem kann beispielsweise ein herkömmliches Gebläse sein, das vor oder nach der Detektionsvorrichtung vorgesehen ist, um die Luft aus dem Rohrleitungssystem anzusaugen.
Diese Gasaufbereitungskomponenten (z.B. Trockner, Heizung, Filter etc.) können in Strömungsrichtung vor oder nach dem Ventil vorgesehen sein. Bei einer Anordnung vor dem Ventil strömt beim Freiblasen das Gas auch durch die Gasaufbereitungskomponenten. Dadurch können auch diese Komponenten beim Freiblasen gereinigt werden.

Ferner ist erfindungsgemäß eine Überdruckquelle vorgesehen, die mit der Ansaugleitung verbindbar ist. Diese Verbindung geschieht insbesondere über das erfindungsgemäße Ventil. Die Überdruckquelle bzw. das von der Überdruckquelle abgegebene gasförmige Medium zum Freiblasen der Ansaugleitung weist einen Druck auf, der größer ist als der Druck in der Ansaugleitung. Dadurch ist gewährleistet, dass das Medium der Überdruckquelle die Leitung freibläst. Insbesondere ist der Druck des Mediums der Überdruckquelle größer als der Druck im Rohrleitungssystem und größer als der Umgebungsdruck. Dadurch wird das gasförmige Medium der Überdruckquelle in die Ansaugleitung gegen die Ansaugrichtung bewegt und die Ansaugleitung wird freigeblasen. Gegebenenfalls wird das gasförmige Medium der Überdruckquelle durch die Ansaugleitung in das Rohrleitungssystem geleitet. Die Ansaugrichtung ist die Strömungsrichtung der Luft des Rohrleitungssystems in der Ansaugleitung, die sich einstellt, wenn das Ansaugsystem die Luft in der Ansaugleitung zur Detektionsvorrichtung fördert.

Beispielsweise beträgt der Druck der Überdruckquelle mehr als 2, 3, 4, 5, 6, 7, oder 8 bar, bevorzugt etwa 8 bar.

Um eine Beschädigung der Detektionsvorrichtung durch den Druckstoß der Überdruckquelle zu verhindern, ist das erfindungsgemäße Ventil derart ausgebildet, dass die Verbindung zwischen der Ansaugleitung und der Detektionsvorrichtung selbsttätig geschlossen wird, wenn die Überdruckquelle auf das Ventil wirkt. Wirkt die Überdruckquelle hingegen nicht auf das Ventil, so wechselt es selbsttätig in eine Position, in der die Ansaugleitung mit der Detektionsvorrichtung verbunden ist, sodass eine Detektion ermöglicht bzw. wieder ermöglicht ist.

Erfindungsgemäß weist das Ventil einen Kolben auf, der entlang einer Bewegungsachse in einem Ventilgehäuse bewegbar, bevorzugt linear bewegbar, ist. Auf eine erste Stirnseite des Kolbens wirkt gegebenenfalls der Druck der Überdruckquelle. Auf die gegenüberliegende Stirnseite des Kolbens wirkt gegebenenfalls ein Druckmittel, wie beispielsweise eine Druckfeder, ein Gummipuffer, eine pneumatische Feder oder eine hydraulische Anordnung. Durch den Druck der Überdruckquelle kann der Kolben verschoben werden. Diese Verschiebung geschieht gegen die Kraft des Druckmittels, Durch die Verschiebung des Kolbens wird das Druckmittel gespannt. Bei Ausbleiben des Drucks der Überdruckquelle drückt somit das Druckmittel den Kolben zurück in seine Ausgangsposition. Gegebenenfalls weist der Kolben einen Anschlag auf, durch den einerseits die Ausgangsposition oder andererseits weitere Positionen definiert sind.

Insbesondere kann das Ventil und der Kolben in drei Stellungen gebracht werden:

In einer Ansaugstellung ist die Ansaugleitung mit der Detektionsvorrichtung verbunden, sodass die Luft bzw. das gasförmige Medium im Regelbetrieb behinderungsfrei bzw. ungehindert von der Ansaugleitung in die Detektionsvorrichtung geleitet werden kann.

Ferner kann das Ventil und der Kolben in eine Freiblasstellung gebracht werden, in der die Überdruckquelle mit der Ansaugleitung verbunden ist. In dieser Stellung ist die Detektionsvorrichtung von der Ansaugleitung getrennt. Der Wechsel des Ventils und des Kolbens von der Ansaugstellung in die Freiblasstellung erfolgt bevorzugt über den Druck der Überdruckquelle, insbesondere angetrieben durch die Druckkraft der Überdruckquelle. Die Rückstellung von der Freiblasstellung in die Ansaugstellung erfolgt bevorzugt über das Druckmittel, insbesondere angetrieben durch die Kraft des Druckmittels.

Zwischen der Ansaugstellung und der Freiblasstellung weisen der Kolben und das Ventil gegebenenfalls eine dritte Stellung - die Sperrstellung - auf. In dieser sind die Komponenten Überdruckquelle, Ansaugleitung und Detektionsvorrichtung voneinander getrennt. Dadurch ist verhindert, dass beim Übergang von der Ansaugstellung in die Freiblasstellung eine Überschneidung gegeben ist, bei der der Druckstoß der Überdruckquelle sowohl in die Ansaugleitung als auch ungewollter Weise in die Detektionsvorrichtung strömt.

Bevorzugt ist der Kolben im Ventilgehäuse dichtend angeordnet. Dies bedeutet, dass entlang der Bewegungsrichtung des Kolbens und insbesondere im Spalt zwischen Kolben und Ventilgehäuse eine Abdichtung erfolgt, sodass durch diesen Spalt im Wesentlichen kein Mediumstransport erfolgt.

Gegebenenfalls umfasst die Rauchdetektionsanordnung eine Rückführleitung, über welche der aus dem Rohrleitungssystem entnommene Teilmassenstrom wieder in das Rohrleitungssystem rückgeführt wird. Die Rückführleitung ist dabei mit der Ansaugvorrichtung und/oder der Detektionsvorrichtung verbunden bzw. diesen Komponenten nachgeschaltet und führt zurück zum Rohrleitungssystem des Gebäudes.

In bevorzugter Weise sind alle Komponenten der Rauchdetektionsanordnung in einem Gehäuse vorgesehen. Somit stellt die gesamte Anordnung eine Einheit dar, die platzsparend ist und einfach an bestehende Rohrleitungssysteme angebaut werden kann.

Als Verbindung wird im Sinne der Erfindung eine Fluidverbindung verstanden. Zwei Komponenten gelten als miteinander verbunden, wenn ein Austausch des gasförmigen Mediums zwischen diesen beiden Komponenten ermöglicht ist. Ein herkömmliches Rohr stellt eine Verbindung zwischen zwei Komponenten her. Durch ein Ventil, das beispielsweise ein Absperrorgan, wie ein Schieber oder ein herkömmliches Ventil sein kann, kann die Verbindung getrennt werden.

Der Freiblaskanal weist bevorzugt eine Querschnittsfläche auf, die kleiner ist, als die Stirnfläche des Kolbens. Z.B. beträgt die Querschnittsfläche des Freiblaskanals die Hälfte, ein Drittel, ein Viertel oder ein Achtel der Stirnfläche des Kolbens.

Bevorzugt ist die Querschnittsfläche der Ansaugleitung größer als die Querschnittsfläche des Freiblaskanals. Z.B. doppelt so groß oder größer.

Beispielsweise beträgt der Durchmesser des Freiblaskanals 10mm oder die Fläche des Freiblaskanals ca. 60 bis 100 mm2, bevorzugt etwa 80mm2.
Beispielsweise beträgt der Durchmesser der Stirnfläche des Kolbens 40mm oder die Fläche der Stirnfläche ca. 800 bis 2000 mm2, bevorzugt etwa 1300mm2. Beispielsweise beträgt der Durchmesser der Ansaugleitung 20mm oder die Fläche der Ansaugleitung ca. 200 bis 600mm2, bevorzugt etwa 400mm2.

Der Unterdruck, der durch das Ansaugsystem erzeugt wird weist insbesondere eine geringe Druckdifferenz zu dem im Rohrleitungssystem herrschenden Druck auf. Die Druckdifferenz muss lediglich ausreichen, um Luft aus dem Rohrleitungssystem zur Detektionsvorrichtung zu fördern.

Der im Rohrleitungssystem herrschende Druck entspricht im Wesentlichen dem Umgebungsdruck, oder einem Druck, der geringfügig kleiner ist, als der Umgebungsdruck. Also etwa 1 bar oder weniger.

Der Druck der Überdruckquelle beträgt beispielsweise mehr als 2, 3, 4, 5, 6, 7, oder 8 bar, bevorzugt etwa 8 bar.

In weiterer Folge wird die Erfindung anhand der dargestellten Ausführungsformen weiter beschrieben.
Fig. 1 zeigt eine schematische Ansicht einer möglichen Ausführungsform der erfindungsgemäßen Rauchdetektionsanordnung.
Fig. 2a zeigt eine schematische Schnittdarstellung eines Ventils in der Ansaugstellung.
Fig. 2b zeigt das Ventil aus Fig. 2a, jedoch in einer Ansicht, in der die versteckten Elemente strichliert eingezeichnet sind.
Fig. 3a zeigt ein Ventil in einer Schnittdarstellung in der Freiblasstellung.
Fig. 3b zeigt dasselbe Ventil wie Fig. 3a, jedoch in einer Ansicht, in der die versteckten Teile strichliert dargestellt sind.
Fig. 4 zeigt eine mögliche Ausführungsform eines Kolbens.
Fig. 5 zeigt eine weitere mögliche Ausführungsform eines Kolbens.
Fig. 6a und 6b zeigen eine weitere mögliche Ausführungsform.

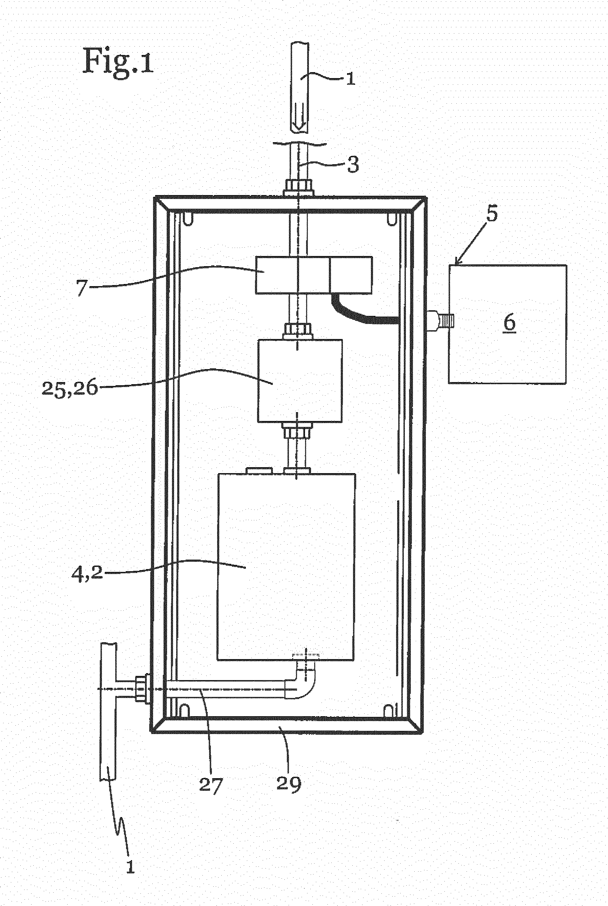

Fig. 1 zeigt die erfindungsgemäße Rauchdetektionsanordnung in einer schematischen Ansicht. Das Gehäuse 29 weist mehrere Anschlüsse auf, die die Gehäusewand durchsetzen. Die Ansaugleitung 3 ist beispielsweise durch die Gehäusewand geführt, um ein gasförmiges Medium wie insbesondere Luft oder Rauchgas von einem Rohrleitungssystem 1 eines Gebäudes an die Detektionsvorrichtung 4 zu leiten. Ein weiterer Anschluss, der das Gehäuse durchsetzt, ist der Anschluss für die Überdruckquelle 6. Die Überdruckquelle 6 kann beispielsweise ein Pufferspeicher für ein Gas und/oder Druckluft oder auch ein Kompressor sein. Die Überdruckquelle 6 ist Teil der Freiblasvorrichtung 5, die mit der Ansaugleitung 3 verbunden ist, um Verunreinigungen wie beispielsweise Staub aus der Ansaugleitung entfernen zu können. Die Ansaugleitung 3 ist mit dem Ansaugsystem 2 verbunden. Dieses ist dazu eingerichtet, einen Unterdruck zu erzeugen, sodass das gasförmige Medium des Rohrleitungssystems 1 zur Detektionsvorrichtung 4 geleitet wird. Im Verlauf der Ansaugleitung 3 bzw. vor der Detektionsvorrichtung 4 - in Strömungsrichtung des gasförmigen Mediums bzw. der Luft des Rohrleitungssystems 1 - ist ein Ventil 7 vorgesehen. Dieses ist dazu eingerichtet, die Ansaugleitung von der Detektionsvorrichtung 4 zu trennen, insbesondere wenn Druckluft von der Überdruckquelle 6 in die Ansaugleitung 3 geleitet ist. Ferner können im Verlauf der Ansaugleitung 3 bzw. vor der Detektionsvorrichtung 4 weitere Komponenten wie beispielsweise ein Filter 25 bzw. eine Trocknungsvorrichtung 26 vorgesehen sein. Diese und weitere Komponenten können dazu eingerichtet sein, die angesaugte Luft derart aufzubereiten, dass eine effiziente Detektion durch die Detektionsvorrichtung 4 ermöglicht ist.

In der vorliegenden Ausführungsform ist eine Rückführungsleitung 27 vorgesehen, die das angesaugte gasförmige Medium nach der Detektionsvorrichtung zurück in das Rohrleitungssystem oder in das Gebäude leitet.

Bei der vorliegenden Ausführungsform wird über die Ansaugleitung 3 bzw. das Ansaugsystem 2 ein Teilmassenstrom oder der gesamte Massenstrom der Luft des Rohrleitungssystems 1 entnommen, durch ein Ventil 7 und gegebenenfalls den Filter 25 oder die Trocknungsvorrichtungen 26 in die Detektionsvorrichtung 4 geleitet. In der Detektionsvorrichtung 4 wird festgestellt, ob sich Rauchgasbestandteile in dem abgezweigten Teilmassenstrom befinden. Ist dies der Fall, so wird an eine Steuerzentrale ein Signal weitergeleitet, das dann beispielsweise einen Alarm auslösen kann. Der entnommene Teilmassenstrom wird gegebenenfalls über die Rückführungsleitung 27 zurück in das Rohrleitungssystem 1 geleitet.

Bevorzugt geschieht die Abzweigung des Teilmassenstroms kontinuierlich. Dadurch können sich in der Ansaugleitung Staub oder ähnliche Partikel ansammeln. Dies kann zur Verlegung der Ansaugleitung bzw. zur Verringerung des Durchflusses führen. Um dies zu vermeiden, ist die Freiblasvorrichtung 5 vorgesehen. Diese umfasst die Überdruckquelle 6, die über eine Leitung mit dem Ventil 7 verbunden ist. Gesteuert wird die Überdruckquelle bzw. der Massenstrom der Überdruckquelle bevorzugt über ein steuerbares Ventil wie beispielsweise ein Magnetventil. Über dieses kann der Überdruck der Überdruckquelle an das Ventil und weiter in die Ansaugleitung 3 geleitet werden, um diese freizublasen.

Während dieses Freiblasens ist es von großer Wichtigkeit, dass die Verbindung zur Detektionsvorrichtung unterbrochen ist, wie anhand der folgenden Figuren weiter beschrieben wird:

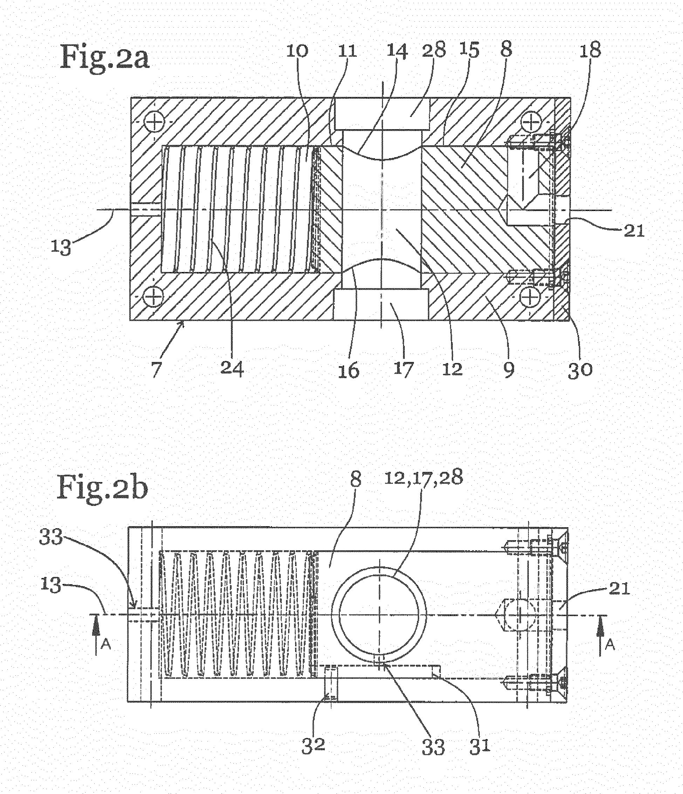

Fig. 2a zeigt eine mögliche Ausführungsform eines Ventils 7 in der Ansaugstellung. Das Ventil 7 umfasst einen Kolben 8, der in einem Ventilgehäuse 9 verschieblich angeordnet ist. Dazu weist das Ventil 7 eine Kolbenöffnung 10 auf, in welcher der Kolben 8 angeordnet ist. Zwischen dem Kolben 8 und dem Ventilgehäuse 9 ist an der Innenseite der Kolbenöffnung der Kolbenspalt 11 vorgesehen. Bevorzugt ist dieser derart schmal ausgeführt, dass ein Gastransport im Wesentlichen verhindert ist. Der Kolben 8 kann entlang der Bewegungsachse 13 verschoben werden. Der Kolben 8 umfasst einen Lüftungskanal 12. Dieser verläuft im Wesentlichen quer zur Bewegungsachse 13 und tritt an einer ersten Stelle 14 der Mantelfläche 15 in den Kolben 8 ein und tritt an einer zweiten Stelle 16 der Mantelfläche wieder aus dem Kolben aus, sodass der Kolben durch den Lüftungskanal 12 durchsetzt ist. Das Ventilgehäuse 9 weist eine erste Lüftungsöffnung 28 sowie eine zweite Lüftungsöffnung 17 auf. In der dargestellten Lüftungsstellung ist der Kolben 8 derart positioniert, dass der Lüftungskanal 12 die erste Lüftungsöffnung 28 mit der zweiten Lüftungsöffnung 17 verbindet und ein Transport des gasförmigen Mediums des Rohrleitungssystems quer durch das Ventil ermöglicht ist. Die erste Lüftungsöffnung 28 ist bevorzugt mit der Ansaugleitung verbunden.

Die zweite Lüftungsöffnung 17 ist in weiterer Folge mit der Detektionsvorrichtung verbunden. In dieser Stellung ist ein Transport der Luft des Rohrleitungssystems zur Detektionsvorrichtung ermöglicht.

Der Kolben 8 ist durch ein Druckmittel 24 im Wesentlichen in dieser Stellung gehalten. Das Druckmittel 24 ist in der vorliegenden Ausführungsform eine Druckfeder. Pneumatische Federn oder andere elastische Körper können ebenfalls eingesetzt werden. Dabei ist der Kolben 8 über das Druckmittel 24 an den Ventildeckel 30 gedrückt, der dadurch als Anschlag wirkt. Ferner kann der Ventildeckel 30 dazu geeignet und/oder eingerichtet sein, die Überdruckquelle 6 an das Ventil anzuschließen oder auch ein Magnetventil anzuschließen, über das die Zuführung des gasförmigen Mediums der Überdruckquelle 6 ermöglicht ist.

Fig. 2b zeigt dasselbe Ventil, jedoch in einer Darstellung, die im Wesentlichen einer 90° verdrehten Ansicht der Ansicht der Fig. 2a entspricht. Der Kolben 8 befindet sich in der Ansaugstellung. Die erste Lüftungsöffnung 28 bzw. die zweite Lüftungsöffnung 17 sind im Wesentlichen fluchtend mit dem Lüftungskanal 12 angeordnet.

Im Kolben 8 ist ferner eine Führungsnut 31 vorgesehen, die im Wesentlichen entlang des Mantels 15 und parallel zur Bewegungsachse 13 verläuft. In die Führungsnut 31 ragt ein Führungsmittel 32, das beispielsweise bolzenförmig ausgebildet ist und mit dem Ventilgehäuse 9 verbunden ist. Durch diese Anordnung ist eine Verschiebung des Kolbens 8 entlang der Bewegungsachse 13 ermöglicht, wobei gleichzeitig eine Verdrehung des Kolbens um die Bewegungsachse 13 verhindert ist.

Ferner sind in dieser Darstellung zwei Entlastungsöffnungen 33 eingezeichnet. Es können beide eingezeichneten Entlastungsöffnungen 33 oder auch nur eine oder keine dieser Entlastungsöffnungen vorgesehen sein. Sie dienen in der vorliegenden Ausführungsform dazu, bei einer Verschiebung des Kolbens, die dadurch verdrängte oder angesaugte Luft abzuführen. Die verdrängte Luft kann entweder nach außen, in den Außenbereich des Ventilgehäuses oder auch in den Lüftungskanal 12 geführt sein. In letzterem Fall wird die Luft dabei über die Führungsnut 31 zu einer Bohrung geführt, die in den Lüftungskanal 12 mündet. Die Entlastungsöffnungen 33 haben bevorzugt einen kleinen Querschnitt, der insbesondere kleiner ist als der Querschnitt des Lüftungskanals 12 und/oder des Freiblaskanals 18.

Ein weiterer Vorteil des ausschließlichen Vorsehens einer Entlastungsöffnung 33, die in den Lüftungskanal 12 mündet ist, dass das Ansaugen von Fremdluft verhindert ist. Bei der Entlastungsöffnung 33, die sich bis nach außen erstreckt, ist bei der Rückbewegung des Kolbens ein Ansaugen der Umgebungsluft ermöglicht. Dies könnte die Messergebnisse der Detektionsvorrichtung verfälschen. Somit ist gemäß einer bevorzugten Ausführungsform der Druckausgleich durch die Verdrängung des bewegten Kolbens lediglich in den Lüftungskanal 12 ermöglicht. Eine Entlüftungsöffnung 33, die nach außen führt, wäre bei dieser Ausführungsform nicht vorgesehen.

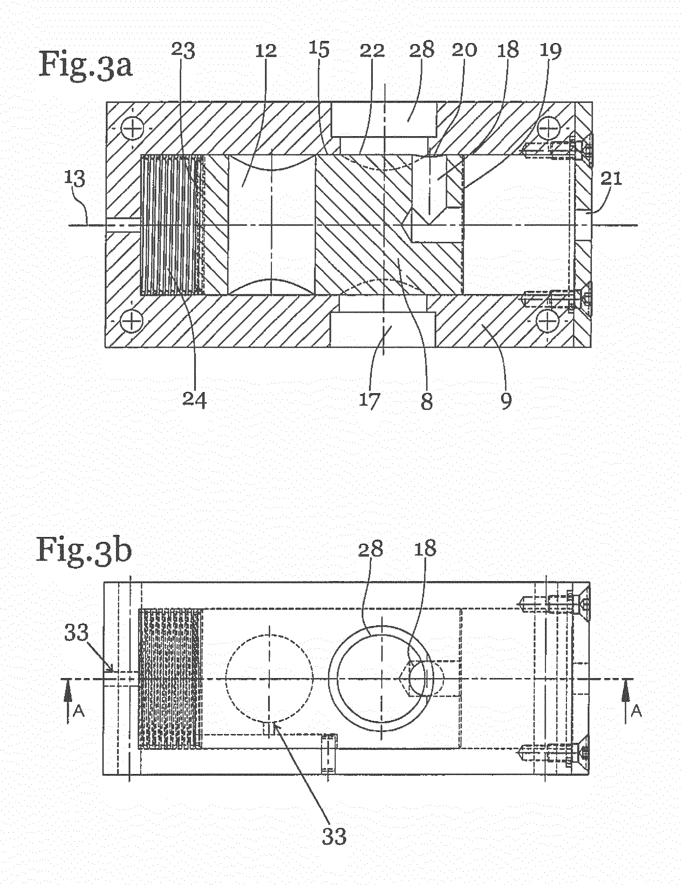

Fig. 3a zeigt ein Ventil, insbesondere das Ventil aus Fig. 2a, in der Freiblasstellung, bzw. im Übergang zwischen der Ansaugstellung in die Freiblasstellung. Der Kolben 8 ist wiederum im Wesentlichen linear in einem Ventilgehäuse 9 geführt. In der gezeigten Stellung ist der Kolben gegen die Kraft des Druckmittels 24 verschoben. Dieses ist zusammengedrückt und wirkt gegen die zweite Stirnseite 23 des Kolbens 8. Der im Kolben 8 vorgesehene Lüftungskanal 12 ist in dieser Stellung durch das Ventilgehäuse 9 verschlossen. Die erste Lüftungsöffnung 28 sowie die zweite Lüftungsöffnung 17 sind nicht mit dem Lüftungskanal 12 verbunden. Der Kolben 8 umfasst einen Freiblaskanal 18. Dieser erstreckt sich von der ersten Stirnseite 19 des Kolbens 8 bis zu einer dritten Stelle 20 der Mantelfläche 15 des Kolbens 8. Somit ist das gasförmige Medium der Überdruckquelle über den Freiblaskanal 18 in die erste Lüftungsöffnung 28 und weiter in die nicht dargestellte Ansaugleitung 3 geleitet. Zwischen dem Austritt des Lüftungskanals 12 und des Freiblaskanals 18 an der Mantelfläche 15 ist ein Steg 22 vorgesehen. Dieser Steg 22 ist in Achsrichtung 13 breiter ausgeführt, als der Durchmesser der ersten Lüftungsöffnung 28. Dies bewirkt den Effekt, dass wenn der Kolben 8 von der Lüftungsstellung, dargestellt in Fig. 2a, in die Freiblasstellung, dargestellt in der Fig. 3a wechselt, zwischen den beiden Stellungen eine Sperrstellung durchläuft. In dieser Sperrstellung sind durch den Kolben 8 sowohl die erste Lüftungsöffnung 28 als auch die zweite Lüftungsöffnung 17 verschlossen. Dadurch wird verhindert, dass eine Überschneidung der beiden Kanäle 12 und 18 auftritt, bei der Überdruck der Überdruckquelle über den Lüftungskanal 12 zur Detektionsvorrichtung 4 geleitet wird.

Der Kolben 8 ist durch den Druck des gasförmigen Mediums der Überdruckquelle gegen die Kraft des Druckmittels 24 verschoben. Wird die Überdruckquelle jedoch vom Ventil getrennt, so wird der Kolben 8 über das Druckmittel 24 selbsttätig in die Ansaugstellung zurück bewegt. Dieser besonders einfache und wartungsfreie Aufbau stellt einen wichtigen Kern der Erfindung dar. Um die Bewegung durch die Überdruckquelle zu ermöglichen, kann der Freiblaskanal 18 derart ausgebildet sein, dass der Druck an der ersten Stirnseite 19 größer ist, als im Freiblaskanal 18 und bevorzugt auch größer als in der Ansaugleitung 3 bzw. im Rohrleitungssystem 1. Dies wird beispielsweise dadurch erreicht, dass der Freiblaskanal 18 einen geringeren Durchmesser aufweist, als die Kolbenöffnung 10. Dadurch wirkt ein Teil des Freiblaskanals 18 als Drossel. Der Staudruck bewegt den Kolben gegen die Kraft des Druckmittels 24 in die Sperrstellung und weiter in die Freiblasstellung. Ferner ist die durch die Überdruckquelle auf die erste Stirnseite wirkende Druckkraft größer als die auf die zweite Stirnseite wirkende Kraft des Druckmittels, um eine Verschiebung des Kolbens von der Ansaugstellung in die Freiblasstellung zu bewirken. In der Freiblasstellung mündet der Freiblaskanal 18 in die Ansaugleitung. Diese Verbindung geschieht über die erste Lüftungsöffnung 28. Der Kolben wird in der Freiblasstellung durch ein Kräftegleichgewicht gehalten, solange der Druck der Überdruckquelle auf den Kolben wirkt. Die Gegenkraft kann beispielsweise durch das Druckmittel oder durch einen Anschlag aufgebracht sein.

Fig. 3b zeigt eine Ansicht des Ventils aus Fig. 3a, die im Wesentlichen 90° verdreht ist. Der Freiblaskanal 18 ist überschneidend mit der ersten Lüftungsöffnung 28 angeordnet. Dadurch kann der Überdruck der Überdruckquelle in die Ansaugleitung 3 geleitet werden.

Wiederum sind zwei alternative Möglichkeiten der Entlastungsöffnungen 33 vorgesehen. Gegebenenfalls können auch beide dargestellten Entlastungsöffnungen 33 vorgesehen sein.

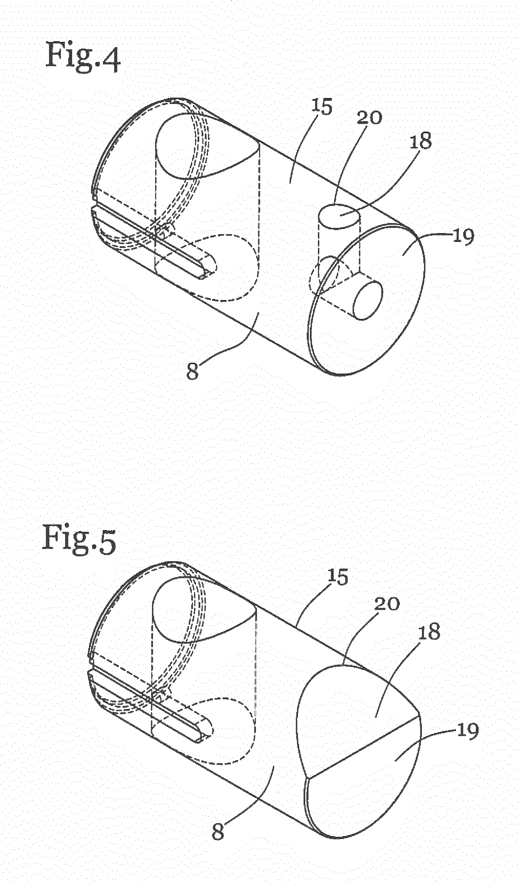

Fig. 4 zeigt eine mögliche Ausgestaltung des Kolbens 8 und insbesondere des Freiblaskanals 18. Fig. 4 zeigt jene Ausführungsform, die auch in den Figuren 2 und 3 dargestellt ist. Dabei erstreckt sich der Freiblaskanal 18 von der ersten Stirnseite 19 des Kolbens 8 ins Innere des Kolbens und tritt an einer dritten Stelle 20 der Mantelfläche 15 wieder aus dem Kolben 8 aus. Beispielsweise kann dieser Kanal 18 durch einen eingelegten Kern bei der Gussfertigung oder durch zwei Bohrungen erstellt werden. Durch diese Ausgestaltung ist eine Leitung des gasförmigen Mediums von der Überdruckquelle in die Ansaugleitung ermöglicht.

Fig. 5 zeigt eine alternative Ausführungsform des Kolbens, die sich insbesondere durch die Ausgestaltung des Freiblaskanals 18 unterscheidet. Wiederum verläuft der Freiblaskanal von der ersten Stirnseite 19 des Kolbens 8 zur Mantelfläche 15 bzw. zu einer dritten Stelle 20 der Mantelfläche 15 des Kolbens. Jedoch ist der Freiblaskanal 18 nicht, wie in Fig. 4 dargestellt, als innenliegender Kanal ausgeführt, sondern ist durch eine Abschrägung des Kolbens 8 gegeben.

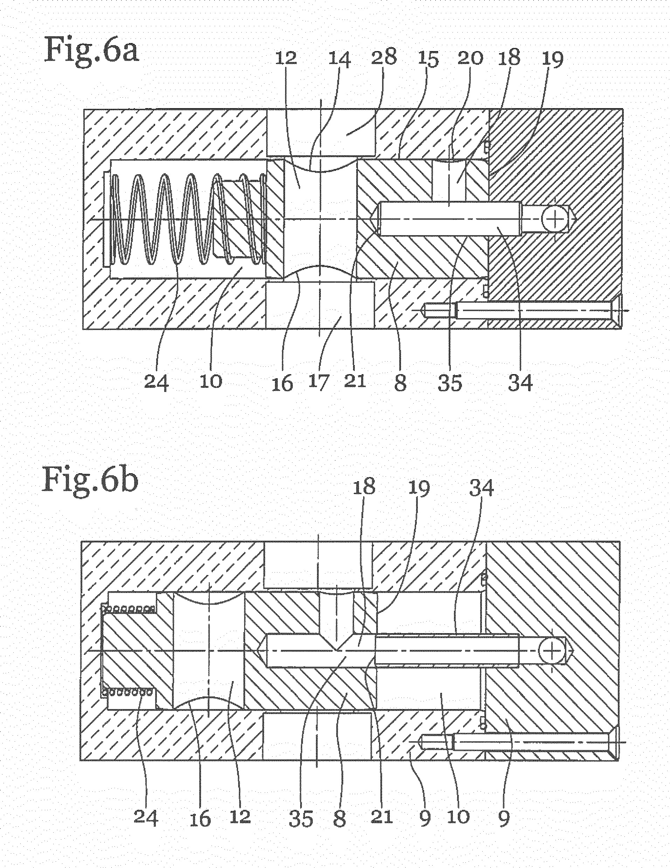

Die Fig. 6a und 6b zeigen Details einer weiteren Ausführungsform der vorliegenden Erfindung, wobei Fig. 6a eine Schnittdarstellung eines Ventils in der Ansaugstellung zeigt und Fig. 6b dasselbe Ventil wie Fig. 6a zeigt - jedoch in der Freiblasstellung.

Die grundsätzliche Konfiguration des Ventils entspricht jener der Fig. 2a oder 3a, Zusätzlich zu den beschriebenen Elementen der Fig. 2a und 3a sind in der Ausführungsform gemäß den Fig. 6a und 6b ein Freiblasrohr 34 und eine Ausnehmung 35 vorgesehen.

Das Freiblasrohr 34 ragt vom Gehäuse 9 in die Kolbenöffnung 10, in der der Kolben verschiebbar gelagert ist. Fig. 6a zeigt das Ventil in der Ansaugstellung. In dieser Stellung ragt das Freiblasrohr 34 in die Ausnehmung 35 des Kolbens 8. Das Freiblasrohr 34 und die Ausnehmung 35 sind dazu im Wesentlichen koaxial ausgerichtet. Insbesondere ist das Freiblasrohr 34 derart ausgestaltet, dass es zumindest teilweise in die Ausnehmung 35 passt. Die Ausnehmung 35 ist bevorzugt an der ersten Stirnseite 19 des Kolbens 8 vorgesehen und erstreckt sich von der ersten Stirnseite 19 des Kolbens 8 in den Kolben 8 hinein. Sowohl die Ausnehmung 35 als auch das Freiblasrohr 34 erstrecken sich bevorzugt entlang der Bewegungsachse 13.

Die Ausnehmung 35 bildet zumindest teilweise den Freiblaskanal 18. In der Ansaugstellung der Fig. 6a ragt das Freiblasrohr 34 in die Ausnehmung 35. Insbesondere ragt es soweit in die Ausnehmung 35, dass der Freiblaskanal 18 verschlossen ist. Der Freiblaskanal 18 ist dabei durch das Freiblasrohr 34 bzw. durch den Mantel des Freiblasrohrs 34 verschlossen. In der Freiblasstellung der Fig. 6b ist der Kolben 8 durch das von der Überdruckquelle zugeführte gasförmige Medium in die Freiblasstellung befördert. In dieser Freiblasstellung mündet der Freiblaskanal 18 in die erste Lüftungsöffnung 28. Das Freiblasrohr 34 befindet sich in der Freiblasstellung gegebenenfalls außerhalb der Ausnehmung 35. Gegebenenfalls ragt das Freiblasrohr 34 jedoch auch in der Freiblasstellung in die Ausnehmung 35. Bevorzugt ist jedoch der Kolben 8 derart verschoben, sodass der Freiblaskanal freigegeben ist, um von der Überdruckquelle kommendes gasförmiges Medium durch den Freiblaskanal 18 zu leiten.

Die Ausnehmung 35 und das Freiblasrohr 34 weisen bevorzugt einen Durchmesser auf, der kleiner ist als der Durchmesser des Kolbens. Durch die verringerte Fläche und die teleskopförmige Anordnung des Freiblasrohrs 34 in der Ausnehmung 35 kann eine verbesserte Bewegungscharakteristik des Kolbens 8 bewirkt werden.
Bevorzugt weisen das Freiblasrohr 34 und die Ausnehmung 35 einen Durchmesser auf, der beispielsweise zwischen einem Fünftel und einem Drittel des Durchmessers des Kolbens liegt.

Darüber hinaus kann in allen Ausführungsformen, wie in den Fig. 6a und 6b dargestellt, ein Stempel angeordnet sein, der bei zusammengedrückter Feder an dem Gehäuse ansteht um einen definierten Anschlag zu bilden.

In weiterer Folge werden beispielhafte Details der Erfindung weiter beschrieben:

### Das Umschaltventil

Das Umschaltventil dient dazu, den Vorgang der Abtrennung des RAS (Rauchansaugsystem) mit anschließender Druckluftbeaufschlagung einerseits elektronikunabhängig zu automatisieren und andererseits eine solche Freiblaseinrichtung deutlich kostengünstiger und kleiner als bisher zu gestalten und, im Falle der automatischen Aktivierung, den Anspruch an die elektronische Steuerung zu verringern, da lediglich ein kleines Magnetventil angesteuert werden muss. Alle nachfolgenden Schalterfordernisse erfolgen rein pneumatisch bzw. mechanisch. Das Umschaltventil besteht z.B. aus einem Gehäuse sowie einem Kolben und einer Rückstellkraft (z.B. einer Feder). Das Gehäuse ist so geformt, dass sich der Kolben im Gehäuse geführt bewegen kann. Der Kolben wird im Gehäuse durch eine geeignete Ausformung am Verdrehen gehindert. Das Gehäuse bietet Vorrichtungen, um sowohl die Rohrleitung als auch die RAS-Zuleitung direkt mit dem Gehäuse luftdicht verbinden zu können.
Das Gehäuse bietet Vorrichtungen, um eine elektronische Ventilsteuereinrichtung mit integriertem Magnetventil luftdicht anbauen zu können.

### Funktion

In der Ruhestellung des Umschaltventils (der Normalbetriebsstellung für den Ansaugbetrieb des RAS) wird der Kolben durch eine Rückstellkraft in seiner Ruhestellung gehalten. In dieser ist eine freie, weitgehend ungehinderte Luftbewegung vom Rohrsystem zum RAS gewährleistet.
Für die Reinigungsfunktion wird am Drucklufteinlass durch eine externe Einrichtung Druckluft zum Einströmen in die Druckkammer gebracht; durch den einseitig am Kolben anstehenden Luftdruck bewegt sich der Kolben gegen die Rückstellkraft, verschließt zuerst den Durchfluss zwischen dem Rohrsystem und dem RAS und gibt danach den Durchfluss der Druckluft in das Rohrsystem frei; er befindet sich dann in seiner Arbeitsstellung bzw. der Freiblasstellung.

### Entlastungsöffnung:

Um die Bewegung des Kolbens nicht durch den Aufbau eines Gegendruckes durch die Volumenverkleinerung in der Federkammer zu behindern sowie um dem Kolben nach Abschalten der Druckluftzufuhr die ungehinderte Rückkehr in die Ruhestellung zu ermöglichen ist eine Entlastungsöffnung vorgesehen; diese Öffnung kann entweder von der Federkammer durch die Gehäusewand in die Umgebung führen oder durch eine Verbindungsöffnung in den RAS-Ansaugkanal des Kolbens führen. Durch die Anordnung dieser Öffnung in den RAS-Ansaugkanal wird verhindert, dass im Ansaugbetrieb geringe Mengen an Fremdluft durch die Entlastungsöffnung angesaugt wird und dadurch das zu messende Medium verdünnt wird. Eine weitere Funktion der Entlastungsbohrung ist, dass sie bei entsprechender Dimensionierung die Aufprallgeschwindigkeit des Kolbens am Gehäuse durch den sich temporär aufbauenden Gaspolster begrenzt.

### Druckluftführung:

Die Druckluftführung von der Druckkammer in das Rohrsystem kann entweder innerhalb des Kolbens durch einen geeigneten Luftführungsdurchgang erfolgen oder direkt aus der Druckkammer in das Rohrsystem indem der Kolben diesen Weg in der Arbeitsstellung freigibt.

### Abdichtung zum Rauchansaugsystem:

Der Kolben wird mittels dem sich einstellenden Gegendruck an die Öffnungsbohrung, die in das RAS führt, gepresst. Durch die Wahl eines elastischen Materials ist an dieser Stelle eine vollständige Abdichtung der Öffnung zum RAS gewährleistet.

### Bezugszeichen

1. Rohrleitungssystem
2. Ansaugsystem
3. Ansaugleitung
4. Detektionsvorrichtung
5. Freiblasvorrichtung
6. Überdruckquelle
7. Ventil
8. Kolben
9. Ventilgehäuse
10. Kolbenöffnung
11. Kolbenspalt
12. Lüftungskanal
13. Bewegungsachse
14. erste Stelle der Mantelfläche
15. Mantelfläche
16. zweite Stelle der Mantelfläche
17. zweite Lüftungsöffnung
18. Freiblaskanal
19. erste Stirnseite des Kolbens
20. dritte Stelle der Mantelfläche
21. Freiblasöffnung
22. Steg
23. zweite Stirnseite des Kolbens
24. Druckmittel
25. Filter
26. Trocknungsvorrichtung
27. Rückführungsleitung
28. erste Lüftungsöffnung
29. Gehäuse
30. Ventildeckel
31. Führungsnut
32. Führungsmittel
33. Entlastungsöffnung
34. Freiblasrohr
35. Ausnehmung

## Patentansprüche

1. Rauchdetektionsanordnung zur Detektion von Rauchgas und/oder Rauchpartikeln in der Luft eines Gebäudes, umfassend:
- ein Ansaugsystem (2) zur Ansaugung von Luft über ein Rohrleitungssystem(1),
- eine Ansaugleitung (3), über die das Rohrleitungssystem (1) mit dem Ansaugsystem (2) verbunden ist,
- eine Detektionsvorrichtung (4), die mit dem Ansaugsystem (2) und/oder der Ansaugleitung (3) verbunden ist, um Rauchgas in der aus dem Rohrleitungssystem (1) angesaugten Luft zu detektieren,
- eine Freiblasvorrichtung (5) mit einer Überdruckquelle (6) zur Einleitung eines gasförmigen Mediums in die Ansaugleitung (3), wobei ein Ventil (7) vorgesehen ist, über das eine Verbindung der Überdruckquelle (6) mit der Ansaugleitung (3) geöffnet oder geschlossen werden kann,
**dadurch gekennzeichnet,**
**dass** das Ventil (7) eine Ansaugstellung aufweist, in der die Detektionsvorrichtung (4) mit dem Ansaugsystem (2) und der Ansaugleitung (3) verbunden ist,
**dass** das Ventil (7) eine Freiblasstellung aufweist, in der die Detektionsvorrichtung (4) von der Ansaugleitung (3) getrennt ist und in der die Überdruckquelle (6) mit der Ansaugleitung (3) verbunden ist,
und **dass**, wenn ein Überdruck von der Überdruckquelle (6) auf das Ventil wirkt, das Ventil (7), angetrieben vom gasförmigen Medium der Überdruckquelle (6), selbsttätig von der Ansaugstellung in die Freiblasstellung wechselt.

2. Rauchdetektionsanordnung nach Anspruch 1, **dadurch gekennzeichnet, dass** das Ventil (7) zwischen der Freiblasstellung und der Ansaugstellung eine Sperrstellung aufweist, in der die Überdruckquelle (6), die Detektionsvorrichtung (4) und die Ansaugleitung voneinander getrennt sind.

3. Rauchdetektionsanordnung nach Anspruch 1 oder 2, **dadurch gekennzeichnet,**
**dass** das Ventil (7) einen Kolben (8) aufweist, der in einem Ventilgehäuse (9) von der Ansaugstellung in die Freiblasstellung bewegbar ist.

4. Rauchdetektionsanordnung nach Anspruch 3, **dadurch gekennzeichnet, dass** das Ventilgehäuse (9) eine Kolbenöffnung (10) aufweist, deren Profil im Wesentlichen dem Profil des Kolbens (8) entspricht, sodass dieser in der Kolbenöffnung (10) geführt ist und dass bevorzugt der Kolbenspalt (11) zwischen Kolbenöffnung (10) und Kolben (8) im Wesentlichen abgedichtet ist, wobei der Kolben im Ventilgehäuse (9) gegebenenfalls linear geführt ist.

5. Rauchdetektionsanordnung nach Anspruch 3 oder 4, **dadurch gekennzeichnet,**
**dass** der Kolben (8) einen Lüftungskanal (12) umfasst, der im Wesentlichen quer zur Bewegungsachse (13) verläuft und an einer ersten Stelle (14) der Mantelfläche (15) in den Kolben (8) eintritt und an einer zweiten Stelle (16) der Mantelfläche (15) austritt, sodass der Kolben (8) durch den Lüftungskanal (12) durchsetzt ist, dass das Ventilgehäuse (9) eine erste Lüftungsöffnung (28) und eine zweite Lüftungsöffnung (17) umfasst, und dass in der Ansaugstellung des Ventils (7) die erste Lüftungsöffnung (28) über den Lüftungskanal (12) des Kolbens (8) mit der zweiten Lüftungsöffnung (17) verbunden ist.

6. Rauchdetektionsanordnung nach einem der Ansprüche 3 bis 5, **dadurch gekennzeichnet, dass** der Kolben (8) einen Freiblaskanal (18) umfasst der sich von der ersten Stirnseite (19) des Kolbens (8) bis zu einer dritten Stelle (20) der Mantelfläche (15) des Kolbens (8) erstreckt,
dass das Ventilgehäuse (9) eine Freiblasöffnung (21) umfasst,
und dass in der Freiblasstellung des Ventils (7) die Freiblasöffnung (21) über den Freiblaskanal (18) des Kolbens (8) mit der ersten Lüftungsöffnung (16) verbunden ist.

7. Rauchdetektionsanordnung nach einem der Ansprüche 3 bis 6, **dadurch gekennzeichnet, dass** zwischen dem Lüftungskanal (12) an der ersten Stelle (14) der Mantelfläche (15) des Kolbens (8) und dem Freiblaskanal (18) an der dritten Stelle (20) der Mantelfläche (15) des Kolbens (8) ein Steg (22) vorgesehen ist, durch welchen die erste Lüftungsöffnung (28) in der Sperrstellung, insbesondere beim Übergang von der Ansaugstellung in die Freiblasstellung, verschlossen ist.

8. Rauchdetektionsanordnung nach einem der Ansprüche 3 bis 7, **dadurch gekennzeichnet, dass** an der zweiten Stirnseite (23) des Kolbens (8) ein Druckmittel (24), bevorzugt ein elastischer Körper wie beispielsweise eine Druckfeder, vorgesehen ist, die insbesondere in der Freiblasstellung gegen den die zweite Stirnseite (23) des Kolbens (8) wirkt, und dass die Druckkraft des Druckmittels (24) in der Ansaugstellung geringer ist, als die durch die Überdruckquelle (6) auf die erste Stirnseite (19) wirkende Druckkraft, sodass sich der Kolben (8) bei angelegter Druckkraft der Überdruckquelle in die Freiblasstellung bewegt und/oder in der Freiblasstellung befindet.

9. Rauchdetektionsanordnung nach einem der Ansprüche 6 bis 8, **dadurch gekennzeichnet, dass** der auf die erste Stirnseite (19) des Kolbens (8) wirkende Druck des gasförmigen Mediums der Überdruckquelle größer ist, als der Druck des gasförmigen Mediums in der Freiblasleitung.

10. Rauchdetektionsanordnung nach einem der Ansprüche 1 bis 9, **dadurch gekennzeichnet, dass** der Druck des gasförmigen Mediums zum Freiblasen der Ansaugleitung im Ventil (7) oder beim Austritt aus dem Ventil (7) größer ist, als der herrschen Luftdruck in der Ansaugleitung (3), sodass das gasförmige Medium der Überdruckquelle zum Freiblasen der Ansaugleitung gegen die Ansaugrichtung in die Ansaugleitung und gegebenenfalls in das Rohrleitungssystem geleitet wird.

11. Rauchdetektionsanordnung nach einem der Ansprüche 6 bis 10, **dadurch gekennzeichnet, dass** das Ventil (7) und/oder der Kolben (8) von der Freiblasstellung selbsttätig in die Ansaugstellung zurückkehrt, wenn die auf die erste Stirnseite (19) des Kolbens (8) wirkende Druckkraft kleiner ist, als die Kraft des Druckmittels.

12. Rauchdetektionsanordnung nach einem der Ansprüche 1 bis 11, **dadurch gekennzeichnet, dass** das Ansaugsystem (2) kontinuierlich Luft und gegebenenfalls in der Luft enthaltenes Rauchgas in die Detektionsvorrichtung (4) fördert, um eine kontinuierliche Rauchgasdetektion zu ermöglichen, dass im Ansaugbereich der Detektionsvorrichtung (4) ein Filter (25), eine Trocknungsvorrichtung (26), eine Kühlvorrichtung oder ähnliche Komponenten vorgesehen sind und/oder dass die in die Detektionsvorrichtung (4) geleitete Luft über eine Rückführungsleitung (27) wieder zurück in das Rohrleitungssystem (1) und/oder das Gebäude geleitet ist.

13. Rauchdetektionsanordnung nach einem der Ansprüche 1 bis 12, **dadurch gekennzeichnet, dass** die Komponenten Ventil (7), Detektionsvorrichtung (4), Ansaugsystem (2) als Einheit in einem Gehäuse angeordnet sind.

14. Rauchdetektionsanordnung nach einem der Ansprüche 1 bis 13, **dadurch gekennzeichnet, dass** der Kolben (8) als runder, insbesondere zylindrischer Kolben ausgeführt ist, der entlang seiner Drehachse bewegbar geführt, jedoch gegen eine Verdrehung gesichert ist.

15. Rauchdetektionsanordnung nach einem der Ansprüche 1 bis 14, **dadurch gekennzeichnet, dass** an der ersten Stirnseite des Kolbens (8) eine entlang der Bewegungsachse (13) in den Kolben (8) verlaufende Ausnehmung (35) oder Bohrung vorgesehen ist, die zumindest einen Teil des Freiblaskanals (18) bildet, dass ein Freiblasrohr (34) vorgesehen ist, das sich vom Gehäuse entlang der Bewegungsachse (13) in die Kolbenöffnung (10) erstreckt und an dessen freien Ende die Freiblasöffnung (21) vorgesehen ist, sodass von der Überdruckquelle (6) kommendes gasförmigen Mediums durch das Freiblasrohr (34) und die Freiblasöffnung (21) Richtung Kolben geleitet werden kann, und dass das Freiblasrohr (34) in der Ansaugstellung in die Ausnehmung (35) ragt.

## Claims

1. Smoke detection assembly for detecting smoke gas and/or smoke particles in the air of a building, comprising:
- an aspiration system (2) for aspirating air via a pipeline system (1),
- an aspiration line (3), the pipeline system (1) being connected thereby to the aspiration system (2),
- a detection apparatus (4) which is connected to the aspiration system (2) and/or the aspiration line (3) in order to detect smoke gas in the air which has been aspirated from the pipeline system (1),
- a purging apparatus (5) having an overpressure source (6) for introducing a gaseous medium into the aspiration line (3), wherein a valve (7) is provided, a connection of the overpressure source (6) to the aspiration line (3) being able to be opened or closed thereby,
**characterised**
**in that** the valve (7) comprises an aspiration position in which the detection apparatus (4) is connected to the aspiration system (2) and the aspiration line (3),
**in that** the valve (7) has a purging position in which the detection apparatus (4) is separated from the aspiration line (3) and in which the overpressure source (6) is connected to the aspiration line (3), and in that when an overpressure from the overpressure source (6) acts on the valve, the valve (7) driven by the gaseous medium of the overpressure source (6) automatically changes from the aspiration position into the purging position.

2. The smoke detection assembly according to Claim 1, **characterised in that** between the purging position and the aspiration position the valve (7) has a locking position in which the overpressure source (6), the detection apparatus (4) and the aspiration line are disconnected from one another.

3. The smoke detection assembly according to Claim 1 or 2, **characterised in that** the valve (7) comprises a piston (8) which is movable in a valve housing (9) from the aspiration position into the purging position.

4. The smoke detection assembly according to Claim 3, **characterised in that** the valve housing (9) comprises a piston opening (10), the profile thereof substantially corresponding to the profile of the piston (8) so that said piston is guided in the piston opening (10), and **in that** preferably the piston gap (11) between the piston opening (10) and the piston (8) is substantially sealed, wherein the piston is optionally guided in a linear manner in the valve housing (9).

5. The smoke detection assembly according to Claim 3 or 4, **characterised in that** the piston (8) comprises a ventilation duct (12) which extends substantially transversely to the movement axis (13) and enters the piston (8) at a first point (14) of the outer surface (15) and emerges at a second point (16) of the outer surface (15) so that the piston (8) is penetrated by the ventilation duct (12), **in that** the valve housing (9) comprises a first ventilation opening (28) and a second ventilation opening (17), and **in that** in the aspiration position of the valve (7) the first ventilation opening (28) is connected to the second ventilation opening (17) via the ventilation duct (12) of the piston (8).

6. The smoke detection assembly according to one of Claims 3 to 5, **characterised in that** the piston (8) comprises a purging duct (18) which extends from the first front face (19) of the piston (8) to a third point (20) of the outer surface (15) of the piston (8),
**in that** the valve housing (9) comprises a purging opening (21)
and **in that** in the purging position of the valve (7) the purging opening (21) is connected via the purging duct (18) of the piston (8) to the first ventilation opening (16).

7. The smoke detection assembly according to one of Claims 3 to 6, **characterised in that** a projection (22) is provided between the ventilation duct (12) at the first point (14) of the outer surface (15) of the piston (8) and the purging duct (18) at the third point (20) of the outer surface (15) of the piston (8), the first ventilation opening (28) being closed thereby in the locked position, in particular in the transition from the aspiration position into the purging position.

8. The smoke detection assembly according to one of Claims 3 to 7, **characterised in that** a pressure means (24), preferably a resilient body such as for example a compression spring, is provided on the second front face (23) of the piston (8), said compression spring acting against the second front face (23) of the piston (8), in particular in the purging position, and **in that** in the aspiration position the compressive force of the pressure means (24) is less than the compressive force acting by the overpressure source (6) on the first front face (19), so that when the compressive force of the overpressure source is applied the piston (8) moves into the purging position and/or is located in the purging position.

9. The smoke detection assembly according to one of Claims 6 to 8, **characterised in that** the pressure of the gaseous medium of the overpressure source acting on the first front face (19) of the piston (8) is greater than the pressure of the gaseous medium in the purging line.

10. The smoke detection assembly according to one of Claims 1 to 9, **characterised in that** the pressure of the gaseous medium for purging the aspiration line is greater in the valve (7) or when emerging from the valve (7) than the prevailing air pressure in the aspiration line (3) so that for purging the aspiration line the gaseous medium of the overpressure source is conducted counter to the aspiration direction into the aspiration line and optionally into the pipeline system.

11. The smoke detection assembly according to one of Claims 6 to 10, **characterised in that** the valve (7) and/or the piston (8) returns automatically from the purging position into the aspiration position when the compressive force acting on the first front face (19) of the piston (8) is lower than the force of the pressure means.

12. The smoke detection assembly according to one of Claims 1 to 11, **characterised in that** the aspiration system (2) continuously conveys air, and optionally smoke gas contained in the air, into the detection apparatus (4) in order to permit a continuous detection of smoke gas,
**in that** a filter (25), a drying apparatus (26), a cooling apparatus or similar components are provided in the aspiration region of the detection apparatus (4) and/or **in that** the air conducted into the detection apparatus (4) is conducted via a return line (27) back into the pipeline system (1) and/or the building.

13. The smoke detection assembly according to one of Claims 1 to 12, **characterised in that** the components: the valve (7), the detection apparatus (4), and the aspiration system (2) are arranged as a unit in a housing.

14. The smoke detection assembly according to one of Claims 1 to 13, **characterised in that** the piston (8) is designed as a round, in particular cylindrical, piston which is movably guided along its rotational axis but is secured against rotation.

15. The smoke detection assembly according to one of Claims 1 to 14, **characterised in that** a recess (35) or bore extending along the movement axis (13) into the piston (8) is provided on the first front face of the piston (8), said recess or bore forming at least one part of the purging duct (18), **in that** a purging pipe (34) which extends from the housing along the movement axis (13) into the piston opening (10) is provided, and the purging opening (21) is provided at the free end of said purging pipe so that gaseous medium coming from the overpressure source (6) may be conducted through the purging pipe (34) and the purging opening (21) in the direction of the piston and **in that** in the aspiration position the purging pipe (34) protrudes into the recess (35).

## Revendications

1. Agencement détecteur de fumée destiné à détecter du gaz de fumée et/ou des particules de fumée dans l'air d'un bâtiment, comprenant :
- un système d'aspiration (2) pour aspirer de l'air par l'intermédiaire d'un système de canalisation (1),
- un conduit aspirant (3), par l'intermédiaire duquel le système de canalisation (1) est relié avec le système d'aspiration (2),
- un dispositif de détection (4) qui est relié avec le système d'aspiration (2) et/ou le conduit aspirant (3) pour détecter du gaz de fumée dans l'air aspiré hors du système de canalisation (1),
- un dispositif de soufflage (5) avec une source de surpression (6) pour introduire un milieu gazeux dans le conduit aspirant (3), une soupape (7) étant prévue, par l'intermédiaire de laquelle une liaison entre la source de surpression (6) et le conduit aspirant (3) peut être ouverte ou fermée,
**Caractérisé**
**en ce que** la soupape (7) comporte une position d'aspiration, dans laquelle le dispositif de détection (4) est relié avec le système d'aspiration (2) et le conduit aspirant (3),
**en ce que** la soupape (7) comporte une position de soufflage, dans laquelle le dispositif de détection (4) est séparé du conduit aspirant (3) et dans laquelle la source de surpression (6) est reliée avec le conduit aspirant (3),
et **en ce que**, lorsqu'une surpression agit à partir de la source de surpression (6) sur la soupape, la soupape (7), entraînée par le milieu gazeux de la source de surpression (6) passe automatiquement de la position d'aspiration dans la position de soufflage.

2. Agencement détecteur de fumée selon la revendication 1, **caractérisé en ce que** la soupape (7) comporte entre la position de soufflage et la position d'aspiration une position de blocage dans laquelle la source de surpression (6), le dispositif de détection (4) et le conduit aspirant sont séparés l'un de l'autre.

3. Agencement détecteur de fumée selon la revendication 1 ou 2, **caractérisé en ce que** la soupape (7) comporte un piston (8) qui dans un corps de soupape (9) est mobile de la position d'aspiration dans la position de soufflage.

4. Agencement détecteur de fumée selon la revendication 3, **caractérisé en ce que** le corps de soupape (9) comporte une ouverture pour piston (10) dont le profil correspond sensiblement au profil du piston (8), de sorte que celui-ci soit guidé dans l'ouverture pour piston (10) et **en ce que** de préférence, l'interstice de piston (11) entre l'ouverture pour piston (10) et le piston (8) soit sensiblement étanchéifié, le piston étant guidé le cas échéant de manière linéaire dans le corps de soupape (9).

5. Agencement détecteur de fumée selon la revendication 3 ou 4, **caractérisé en ce que** le piston (B) comprend un canal d'aération (12) qui s'écoule sensiblement à la transversale de l'axe de déplacement (13) et sur un premier endroit (14) de la surface d'enveloppe (15), pénètre dans le piston (8) et sort sur un deuxième endroit (16) de la surface d'enveloppe (15), de sorte que le piston (8) soit traversé par le canal d'aération (12), **en ce que** le corps de soupape (9) comporte une première ouverture d'aération (28) et une deuxième ouverture d'aération (17), et **en ce que** dans la position d'aspiration de la soupape (7), la première ouverture d'aération (28) est reliée par l'intermédiaire du canal d'aération (12) du piston (8) avec la deuxième ouverture d'aération (17).

6. Agencement détecteur de fumée selon l'une quelconque des revendications 3 à 5, **caractérisé en ce que** le piston (8) comprend un canal de soufflage (18) qui s'étend de la première face frontale (19) du piston (8) jusqu'à un troisième endroit (20) de la surface d'enveloppe (15) du piston (8),
**en ce que** le corps de soupape (9) comprend un orifice de soufflage (21),
et **en ce que** dans la position de soufflage de la soupape (7) l'orifice de soufflage (21) est relié par l'intermédiaire du canal de soufflage (18) du piston (8) avec la première ouverture d'aération (16).

7. Agencement détecteur de fumée selon l'une quelconque des revendications 3 à 6, **caractérisé en ce qu'**entre le canal d'aération (12) sur le premier endroit (14) de la surface d'enveloppe (15) du piston (8) et le canal de soufflage (18) sur le troisième endroit (20) de la surface d'enveloppe (15) du piston (8), il est prévu une barrette (22) par laquelle la première ouverture d'aération (28) est fermée dans la position de blocage, notamment lors du passage de la position d'aspiration dans la position de soufflage.

8. Agencement détecteur de fumée selon l'une quelconque des revendications 3 à 7, **caractérisé en ce que** sur la deuxième face frontale (23) du piston (8), il est prévu un moyen de pression (24), de préférence un corps élastique, comme par exemple un ressort de pression, qui notamment dans la position de soufflage agit contre la deuxième face frontale (23) du piston (8), et **en ce que** la force de pression du moyen de pression (24) dans la position d'aspiration est inférieure à la force de pression exercée par la source de surpression (6) sur la première face frontale (19), de sorte que lorsque la force de pression est appliquée par la source de surpression, le piston (8) se déplace dans la position de soufflage et/ou se trouve dans la position de soufflage.

9. Agencement détecteur de fumée selon l'une quelconque des revendications 6 à 8, **caractérisé en ce que** la pression du milieu gazeux de la source de surpression agissant sur la première face frontale (19) du piston (8) est supérieure à la pression du milieu gazeux dans la position de soufflage.

10. Agencement détecteur de fumée selon l'une quelconque des revendications 1 à 9, **caractérisé en ce que** la pression du milieu gazeux destinée au soufflage du conduit aspirant dans la soupape (7) ou à la sortie hors de la soupape (7) est supérieure à la pression atmosphérique régnant dans le conduit aspirant (3), de sorte que le milieu gazeux de la source de surpression pour le soufflage du conduit aspirant soit dirigé à l'encontre de la direction d'aspiration dans le conduit aspirant et le cas échéant, dans le système de canalisation.

11. Agencement détecteur de fumée selon l'une quelconque des revendications 6 à 10, **caractérisé en ce que** la soupape (7) et/ou le piston (8) retourne automatiquement de la position de soufflage dans la position d'aspiration lorsque la force de pression agissant sur la première face frontale (19) du piston (8) est inférieure à la force du moyen de pression.

12. Agencement détecteur de fumée selon l'une quelconque des revendications 1 à 11, **caractérisé en ce que** le système d'aspiration (2) transporte en continu de l'air et le cas échéant, du gaz de fumée contenu dans l'air dans le dispositif de détection (4), pour permettre une détection continue du gaz de fumée,
**en ce que** dans la zone d'aspiration du dispositif de détection (4) sont prévus un filtre (25), un dispositif de séchage (26), un dispositif de refroidissement ou des composants analogues et/ou **en ce que** l'air dirigé dans le dispositif de détection (4) est redirigé par l'intermédiaire d'un conduit de retour (27) dans le système de canalisation (1) et/ou le bâtiment.

13. Agencement détecteur de fumée selon l'une quelconque des revendications 1 à 12, **caractérisé en ce que** les composants soupape (7), dispositif de détection (4), système d'aspiration (2) sont placés sous la forme d'une unité dans un boîtier.

14. Agencement détecteur de fumée selon l'une quelconque des revendications 1 à 13, **caractérisé en ce que** le piston (8) est conçu sous la forme d'un piston rond, notamment cylindrique, qui est guidé en étant mobile le long de son axe de rotation, qui est toutefois bloqué contre une torsion.

15. Agencement détecteur de fumée selon l'une quelconque des revendications 1 à 14, **caractérisé en ce que** sur la première face frontale du piston (8), il est prévu un évidement (35) ou perçage s'étendant le long de l'axe de déplacement (13) dans le piston (8), qui forme au moins une partie du canal de soufflage (18), **en ce qu'**il est prévu un tube de soufflage (34) qui s'étend à partir du corps, le long de l'axe de déplacement (13) dans l'orifice de piston (10) et sur l'extrémité libre duquel est prévu l'orifice de soufflage (21), de sorte que du milieu gazeux provenant de la source de surpression (6) puisse être dirigé à travers le tube de soufflage (34) et l'orifice de soufflage (21), en direction du piston, et **en ce que** dans la position d'aspiration, le tube de soufflage (34) saillit dans l'évidement (35).
